# EUROPEAN PATENT SPECIFICATION

(11) **EP 4 090 269 B1**
(45) Date of publication and mention of the grant of the patent: **11.06.2025**
(21) Application number: 21705726.4
(22) Date of filing: 18.01.2021
(51) Int. Cl.: A61B 17/34, A61B 18/14, A61B 17/00, A61B 17/29

(54) **ARTICULATING SHAFT OF A SURGICAL DEVICE**
GELENKWELLE EINER CHIRURGISCHEN VORRICHTUNG
ARBRE D'ARTICULATION D'UN DISPOSITIF CHIRURGICAL

(30) Priority: 16.01.2020 US 202062962036 P; 29.01.2020 US 202062967300 P
(43) Date of publication of application: 23.11.2022
(73) Proprietor: SMITH & NEPHEW, INC., Memphis, Tennessee 38116 (US); Smith & Nephew Orthopaedics AG, 6300 Zug (CH)
(72) Inventor: LYNN, Christopher J., Austin, Texas 78735 (US); HAMLIN, Winborne, Austin, Texas 78746 (US); GOODE, Johnson E., Austin, Texas 78733 (US)
(74) Representative: White, Andrew John
(86) International application number: PCT/US2021/013811
(87) International publication number: WO 2021/146677

(56) References cited:
- WO-A2-2006/119495
- US-A1- 2004 199 051
- US-A1- 2005 165 415
- US-A1- 2016 184 039

## Description

### CROSS-REFERENCE TO RELATED APPLICATIONS

This application claims benefit to US Provisional Application No. 62/962036 filed January 16, 2020, titled "Articulating Shaft of a Surgical Device".

This application also claims benefit to US Provisional Application No. 62/967,300, filed January 28, 2020, titled "Articulating Shaft Liner of a Surgical Device".

### FIELD OF THE INVENTION

The present invention relates to the field of surgery and more particularly, to articulating surgical devices and methods of accessing targeted tissue using an articulating device.

### BACKGROUND

This invention pertains to an articulating arthroscopic or endoscopic device. In arthroscopy, the number of portals through the patient's skin are kept to a minimum to reduce patient scarring and improve speed of patient recovery. The locations may not be preferable for accessing all portions of the tissue to be treated within the joint and therefore some devices may be selectively articulable or have a pre-bent or angled end. Portals are preferably limited in size using a 5.0 mm cannula however, restricting the ability for a pre-bent device to be inserted therethrough. This restriction to a smaller cannula size also tends to compromise rigidity of an articulated shaft and typical articulating shafts may flex and not hold their shape when in contact with tissue. The surgeon therefore may have to use a fixed device for treating a tissue in one mode such as bulk tissue removal that may do much of the heavy lifting, and articulating devices may be brought in for precise tissue removal or removal in locations that could not be reached with a fixed device. Therefore, there is a need for a device that may articulate and be sufficiently rigid to hold its shape during use.

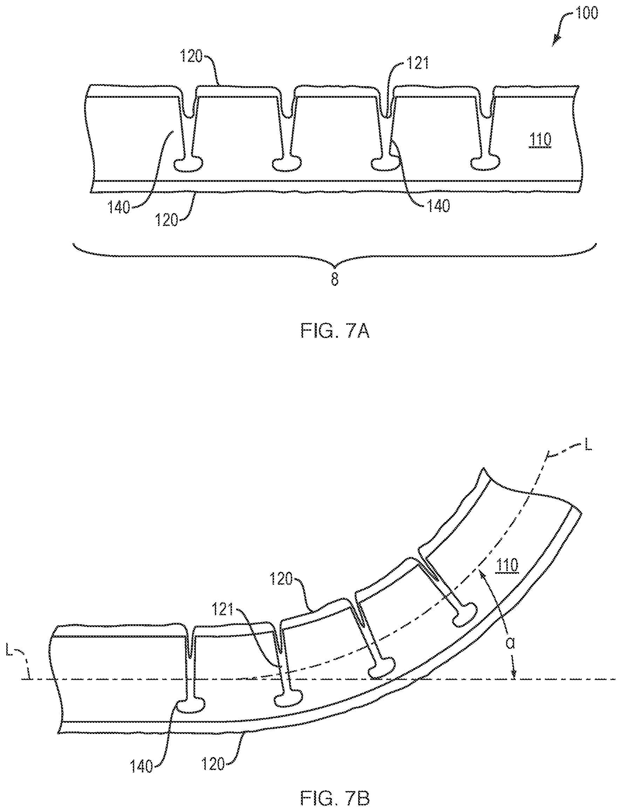

Articulating portions of these shafts may include a plurality of transverse cuts or cutouts through the shaft(s) and arranged along at least a portion of the length of the shaft(s). These cutouts may expand and contract during articulation. In some applications, the plurality of cutouts may be covered by a thin and flexible sheath. Sheath may electrically isolate a portion of the shaft for energy-based devices for example. An example of a device distal end 100 with a sheath 120 may be seen in FIGs 7A-7B. As a first non-limiting example in energy based devices, the tubular shaft 110 may be electrically active and sheath 120 may limit the exposed portion of electrically active surfaces. As a further non-limiting example this sheath 120 may restrict ingress of electrically conductive fluid, present during arthroscopy, through the tubular shaft and limits unintended electrical paths within an energy based device. In grasper style devices the tubular shaft 110 may house moving linkages and the sheath 120 may prevent fluid and debris from entering the tubular shaft 110 via the radial cutouts 140 and interfering with the linkage function. Since this sheath 120 is preferably configured to flex and minimally interfere with articulation, it may also tend to wrinkle and migrate into the radial cutouts 140 with repeated articulation, as the radial cutouts expand and contract as represented in FIG. 7A-7B. More specifically FIG. 7A illustrates a view of distal end 100 of an articulating device in a straight configuration covered by sheath 120. FIG. 7B illustrates a view of distal end 100 in an angled configuration with the sheath 120 with wrinkles 121. FIG. 7A illustrates a cross section of the distal end 100 in the straight configuration with the sheath 120 beginning to migrate between cuts outs 140. FIG. 7B illustrates a cross section of the distal end 100 in a bent or angled configuration with the sheath 120 partially disposed within at least one cutout 140. This migration may hinder articulation and torsional stiffness of the device, in an articulated configuration. Represented in FIG. 7B, wrinkles 121 within at least some of the cutouts 140 limit how closed a cutout may become and therefore device may be limited to a reduced articulation angular offset a. This may also cause the device to require additional force to articulate which can be uncomfortable for the surgeon. There is therefore a need for a means of limiting migration of a sheath 120 into or between radial cutouts along a shaft as the tubular shaft that minimally adds bulk to the shaft or significantly affect ease of articulation.

US 2005/165415 discloses a surgical stapling instrument particularly suited to endoscopic use articulates an end effector (e.g., stapling and severing) that is actuated by a firing bar. An articulation mechanism in an elongate shaft incorporates electrically actuated polymer (EAP) actuators that laterally support the firing bar so that the firing bar is sufficiently constrained to avoid a blow-out, yet is guided without excessive friction and binding.

In US 2016/184039 surgical end effectors are disclosed having angled tissue-contacting surfaces. The end effectors may have a first jaw member that is movable relative to a second jaw member between an open position and a closed position.

US 2004/199051 discloses an articulating shaft includes an outer tubular member having a longitudinal axis and portions defining a slot. An inner tubular member is disposed within the outer member and movable about the axis of the outer member. A wedge carried by the inner member is movable within the slot in an interference fit with the slot portions to articulate the outer member.

WO 2006/119495 discloses a robotic catheter system includes a controller with a master input device. An instrument driver is in communication with the controller and has a guide instrument interface including a plurality of guide instrument drive elements responsive to control signals generated, at least in part, by the master input device. An elongate guide instrument has a base, distal end, and a working lumen, wherein the guide instrument base is operatively coupled to the guide instrument interface.

### BRIEF DESCRIPTION OF THE DRAWINGS

For a detailed description of example embodiments, reference will now be made to the accompanying drawings in which:
FIG. 1 illustrates a view of an articulating device, according to at least some embodiments disclosed herein;
FIG. 2 illustrates an articulating portion of an articulating device, according to at least some embodiments disclosed herein;
FIG. 3A illustrates a side view of cutouts of an outer tube of an articulating portion of an articulating device, according to at least certain embodiments;
FIG. 3B illustrates a cutout of an outer tube of an articulating portion of an articulating device, in an open configuration according to at least certain embodiments;
FIG. 3C illustrates a cutout of an outer tube of an articulating portion of an articulating device in a closed or articulated configuration relative to the open configuration of FIG. 3B;
FIG. 3D illustrates a distal end of an articulating portion of an articulating device in an articulated configuration according to at least certain embodiments;
FIG. 4A and 4B illustrate a cutout with a symmetrical geometry, in a lesser articulated configuration and articulated configuration respectively, and thereby illustrating the unfavorable terracing;
FIG. 5A illustrates alignment of edges of a cutout with an asymmetrical shape, as illustrated in FIG. 3C;
FIG. 5B illustrates alignment of edges of a cutout with an symmetrical shape, as illustrated in FIG. 4;
FIG. 6 illustrates cutouts of an inner tube of an articulating portion according to at least certain embodiments;
FIG. 7A illustrates a tubular member with cutouts and an outer sheath in accordance with some prior art;
FIG. 7B illustrates a cross section of a flexible portion of a shaft with an outer sheath at least partially migrated between cutouts and thereby limiting articulation in accordance with some prior art;
FIG. 8 illustrates a partial assembly of an articulating tubular member with a liner according to at least certain embodiments;
FIG. 9A illustrates a liner with helical cuts according to at least certain embodiments;
FIG. 9B illustrates a liner with helical cuts that has been elongated so as to expand the cuts and form gaps, according to at least certain embodiments;
FIG. 9C illustrates an elongated liner with helical cuts covering an articulable portion of a shaft according to at least certain embodiments;
FIG. 10A illustrates an example electrosurgical wand with an articulable portion according to at least certain embodiments;
FIG. 10B illustrates a side view of the example electrosurgical wand distal end illustrated in FIG. 10A, with portions removed for illustrations purposes; and
FIG. 10C illustrates a cross section of the shaft of the example electrosurgical wand distal end illustrated in FIGS. 10A and 10B.

### SUMMARY

The invention is defined in the independent claim and other embodiments are listed in the dependent claims. No methods are claimed.

Generally, this disclosure describes a surgical device having an articulating tubular member with cuts or slots therethrough, configured to direct articulation. The device may be sized to fit down a 5.0 mm cannula, and articulate to enable access to joint anatomies. Articulation may be achieved by a flexible portion including at least one cutout that is configured to provide improved shaft bending strength and torsional rigidity when in the fully articulated configuration compared to other articulating devices.

This disclosure also describes an articulating surgical device that includes a thin flexible sheath covering at least some of the cutouts. A sleeve liner that may include angular cuts may be coaxially disposed over the cutouts to limit migration of the thin flexible sheath into the radial cutouts during articulation. The liner may define a thin-walled, high melting point tubing that fits between the insulation sheath and metallic shaft that stretches or slides when the device articulates. The surgical device may be an RF device wherein the tubular member may be electrically coupled as an electrode and wherein the sheath limits exposure of the tubular external surface and thereby the electrode location and size.

A first example embodiment of a surgical instrument may include a handle and an elongate shaft assembly extending distally from the handle. The elongate shaft has a longitudinal axis. The elongated shaft assembly is articulable between a fully flexed configuration and a lesser flexed configuration. The elongated shaft assembly includes a tubular member including a flexible or articulable portion with a first preferential bending direction. There is also an elongate member coaxially disposed within the tubular member and directly coupled to the tubular member at a location distal the flexible portion. Axial tension on the elongate member may articulate the shaft assembly. The flexible portion is defined by a plurality of cutouts, through the thickness of the tubular member. Each cutout defines a cutout longitudinal axis. Each cutout includes a tapered opening portion defined by a pair of linear edges facing each other. Linear edges may be defined by which side of the cutout they are located. For example, the pair of linear edges may be termed a proximal linear edge and a distal linear edge. At least one of the plurality of cutouts define an asymmetrical shape about its corresponding cutout longitudinal axis, the asymmetric shape configured to increase a circumferential length of contact between the pair of linear edges, when the flexible portion is in the fully flexed configuration.

In some embodiments, at least one of the cutouts defines a keyhole shape with a bulbous closed end, the bulbous closed end asymmetrical about its cutout longitudinal axis. The bulbous closed end may define a proximal concave surface and a distal concave surface, which are continuous with each other, and wherein the distal concave surface defines a tighter radius of curvature than the proximal concave surface. The bulbous closed end may define a maximum axial width that is less than 50% larger than a maximum axial length of tapered opening portion when the instrument is in a lesser flexed configuration. In the fully flexed configuration, the proximal linear edge and distal linear edge of each of the plurality of cutouts may align with each other to form a smooth curved shaft surface, free of terracing. The cutout asymmetrical shape may be configured to rotate the distal edge towards the proximal edge so that they align once engaged. The plurality of cutouts tapered opening portions each define an angular opening when the shaft is in a less articulated configuration, and in a neutral or non-articulated configuration a distal-most cutout of the plurality of cutouts has a smaller angular opening than all of the remaining plurality of cutouts.

Another embodiment of a surgical instrument disclosed herein may include a handle and an elongate shaft assembly extending distally from the handle. The elongated shaft assembly includes a tubular member including an articulable portion defined by at least one cutout through a wall of the tubular member. An elongate member is also coaxially located along the tubular member and coupled to the tubular member at a position distal to the articulable portion. Actuation of the elongate member flexes the articulable portion and angularly offsets a distal end of the tubular member relative to a proximal end of the elongate shaft. The at least one cutout defines a longitudinal axis transverse a shaft longitudinal axis, wherein the at least one cutout defines an asymmetrical shape about the cutout longitudinal axis. The asymmetrical shape is shaped to increase a length of circumferential contact between a proximal and distal circumferential edge of each cutout when the distal end is flexed at a maximum angular offset.

In some embodiments, the at least one cutout defines a keyhole shape with a bulbous closed end, the bulbous closed end defining a concave proximal surface and a concave distal surface facing the concave proximal surface and different in radius of curvature to the concave proximal surface. The concave proximal surface may define a larger radius of curvature than the concave distal surface. The asymmetrical shape may be configured to form a continuous uninterrupted curve along the articulable portion when the distal end is flexed at the maximum offset angle. The at least one cutout may comprise at least two cutouts, each of the at least two cutouts defining an angular opening and wherein when angular opening of the distal-most cutout of the at least two cutouts is formed with a smaller angular opening than all remaining cutouts.

Another example additional surgical instrument embodiment is disclosed herein, including a handle and an elongated shaft assembly extending distally from the handle. The elongate shaft assembly includes a tubular member having an articulable portion defined by a plurality of axially spaced cutouts through the tubular member. The elongate shaft assembly also includes a sheath, coaxial with the tubular member and covering the tubular member including covering the articulable portion. The elongate shaft member also includes a liner disposed between the sheath and tubular member, along the articulable portion. The liner includes at least one discontinuity through a thickness of the liner, configured to permit the articulable portion to articulate while concomitantly blocking migration of the sheath into the cutouts.

In some embodiments, the discontinuity may include a helical cut around and along a portion of the liner. The discontinuity may comprise a helical cut having an axial gap length, the axial gap length configured to increase liner flexibility while concomitantly blocking migration of the sheath into the radial cutouts. The axial gap length may be formed by axially stretching or extending the liner. The tubular member may be electrically conductive and the sheath may be an electrical insulator, configured to limit an exposed electrically conductive outer surface of the tubular member. The liner may be a Nylon sleeve. Substantially each revolution of the helical cut may occur within a respective distance along a length of the articulable portion including at least two of the plurality of cutouts. Substantially each revolution of the helical cut may occur within a respective distance along an axial length of the articulable portion so as to cover at least three of the plurality of radial cut outs.

An additional example embodiment is disclosed that may include a handle and an elongated shaft assembly extending distally from the handle. The elongated shaft assembly may include a tubular member with a flexible portion defined by a plurality of radial cutouts through the tubular member, axially spaced along the tubular member. The radial cutouts may open and close while flexing. The elongated shaft assembly may also include a sheath coaxial with the tubular member and covering the tubular member including covering the flexible portion. The elongated shaft assembly may also include a sleeve or liner coaxially disposed between the sheath and tubular member and along the flexible portion. A spiral cut may extend along and around a portion of the sleeve, the spiral cut and sleeve generally configured to permit the flexible portion of the tubular member to articulate, wherein the radial cutouts expand and contract while concomitantly limiting migration of the sheath into the radial cutouts.

In some example embodiments, the sleeve may be stretched or formed to include at least one elongate axial gap. This may be formed by stretching the sleeve such that the spiral cut edges move away from each other. The axial gap may be configured to increase sleeve flexibility while maintaining a block for migration of the sheath into the radial cutouts. In some example embodiments, the tubular member is electrically conductive and the sheath is an electrical insulator, configured to limit an exposed electrically conductive outer surface of the tubular member. In some example embodiments, the sleeve comprises Nylon.. In some example embodiments, substantially each revolution of the spiral cut occurs within a respective distance along an axial length of the flexible portion of three of the plurality of radial cutouts. In some example embodiments substantially each revolution of the spiral cut occurs within a respective distance along an axial length of the flexible portion of two of the plurality of radial cut outs.

A further example embodiment of a surgical instrument may include a handle and an elongated shaft assembly extending distally from the handle. The elongated shaft assembly may include a tubular member having a flexible portion defined by a plurality of transverse cutouts through the tubular member, axially spaced along the tubular member. The elongated shaft assembly may also include a sheath coaxial with the tubular member and covering the flexible portion and a sleeve disposed between the sheath and tubular member and along the flexible portion. The sleeve may flex, stretch, and permit the flexible portion of the tubular member to articulate while concomitantly limiting migration of the sheath in between the transverse cutouts.

In some example embodiments, the tubular member may be electrically conductive and the sheath is an electrical insulator, configured to limit an exposed electrically conductive outer surface of the tubular member. In some example embodiments, the sleeve may define a thin-walled high temperature sleeve. In some example embodiments, the sleeve may include a plurality of incisions orientated at an angle to a longitudinal axis of the sleeve to increase flexibility of the sleeve while concomitantly limiting migration of the sheath in between the transverse cutouts. In some example embodiments, the sleeve may include a single elongate helical cut configured to increase flexibility of the sleeve while concomitantly limiting migration of the sheath in between the transverse cutouts. In some example embodiments each revolution of the single helical cut may occurs within a respective distance along a length of the flexible portion of three of the plurality of transverse cuts. In some example embodiments, each revolution of the helical cut occurs within a respective distance along the length of the flexible portion of two of the plurality of transverse cutouts. In some example embodiments, the helical cut may include axial openings to further increase flexibility of the sleeve while concomitantly limiting migration of the sheath in between the radial cutouts.

### NOTATION AND NOMENCLATURE

Certain terms are used throughout the following description and claims to refer to particular system components. As one skilled in the art will appreciate, companies that design and manufacture electrosurgical systems may refer to a component by different names. This document does not intend to distinguish between components that differ in name but not function.

In the following discussion and in the claims, the terms "including" and "comprising" are used in an open-ended fashion, and thus should be interpreted to mean "including, but not limited to ...." Also, the term "couple" or "couples" is intended to mean either an indirect or direct connection. Thus, if a first device couples to a second device, that connection may be through a direct connection or through an indirect connection via other devices and connections.

Reference to a singular item includes the possibility that there are plural of the same items present. More specifically, as used herein and in the appended claims, the singular forms "a," "an," "said" and "the" include plural references unless the context clearly dictates otherwise. It is further noted that the claims may be drafted to exclude any optional element. As such, this statement serves as antecedent basis for use of such exclusive terminology as "solely," "only" and the like in connection with the recitation of claim elements, or use of a "negative" limitation. Lastly, it is to be appreciated that unless defined otherwise, all technical and scientific terms used herein have the same meaning as commonly understood by one of ordinary skill in the art to which this invention belongs. "Ablation" shall mean removal of tissue based on tissue interaction with a plasma.

"Mode of ablation" shall refer to one or more characteristics of an ablation. Lack of ablation (i.e., a lack of plasma) shall not be considered an "ablation mode."

"Active electrode" shall mean an electrode of an electrosurgical wand which produces an electrically-induced tissue-altering effect when brought into contact with, or close proximity to, a tissue targeted for treatment.

"Return electrode" shall mean an electrode of an electrosurgical wand which serves to provide a current flow path for electrical charges with respect to an active electrode, and/or an electrode of an electrical surgical wand which does not itself produce an electrically-induced tissue-altering effect on tissue targeted for treatment.

### DETAILED DESCRIPTION

The following discussion is directed to various embodiments.

Although one or more of these embodiments may be preferred, the embodiments disclosed should not be interpreted, or otherwise used, as limiting the scope of the disclosure.

In addition, one skilled in the art will understand that the following description has broad application, and that the invention is defined by the claims.

Before the present invention is described in detail, it is to be understood that this invention is not limited to particular variations set forth herein as various changes or modifications may be made to the invention described without departing from the scope of the invention. As will be apparent to those of skill in the art upon reading this disclosure, each of the individual embodiments described and illustrated herein has discrete components and features which may be readily separated from or combined with the features of any of the other several embodiments without departing from the scope of the present invention. In addition, many modifications may be made to adapt a particular situation, material, composition of matter, process, process act(s) or step(s) to the objective(s),spirit or scope of the present invention.

The invention is defined by the appended claims.

Example methods which do not form part of the claimed invention recited herein may be carried out in any order of the recited events, which is logically possible, as well as the recited order of events. Furthermore, where a range of values is provided, it is understood that every intervening value, between the upper and lower limit of that range and any other stated or intervening value in that stated range is encompassed within the disclosure.

Reference to a singular item, includes the possibility that there are plural of the same items present. More specifically, as used herein and in the appended claims, the singular forms "a," "an," "said" and "the" include plural referents unless the context clearly dictates otherwise. It is further noted that the claims may be drafted to exclude any optional element. As such, this statement is intended to serve as antecedent basis for use of such exclusive terminology as "solely," "only" and the like in connection with the recitation of claim elements, or use of a "negative" limitation. Last, it is to be appreciated that unless defined otherwise, all technical and scientific terms used herein have the same meaning as commonly understood by one of ordinary skill in the art to which this invention belongs.

The inventors have recognized that it may be desirable to provide an articulable elongated shaft assembly with sufficient rigidity in an articulated configuration. Such rigidity may help to maintain a distal tip of the elongated shaft assembly in a desired position and/or orientation during a surgical procedure and/or avoid excessive deflection of the shaft assembly when a force is applied to the distal tip. For example, the distal tip may be pressed into contact with a surface when deploying a fastener into tissue, and the rigidity of the elongated shaft assembly may limit the deflection of the tip to be less than a desired threshold deflection for a predetermined force applied to the distal tip. As a further example the distal tip may be used to elevate or move tissue to gain better access and electrosurgically treat a target tissue, and the rigidity of the elongated shaft assembly may limit the deflection of the tip while elevating tissue.

As used herein, the term "distal direction" within a surgical device may refer to a direction that extends along a longitudinal axis of the surgical device towards a distal end of the surgical device where a desired operation is performed. Correspondingly, a "proximal direction" may refer to a direction that is directed in an opposite direction relative to the distal direction such that it may be directed along the longitudinal axis of the surgical device away from the surgical device's distal end where the desired operation is performed.

According to some embodiments, an elongated shaft assembly extends distally from a handle of a surgical instrument. The elongated shaft assembly includes an articulable portion that may articulate in at least one direction between a first position, which may correspond to a non-articulated configuration, to a second position, which may correspond to a fully articulated configuration in which the distal tip is oriented at an angle (e.g., an articulation angle) relative to a portion of the elongated shaft assembly located proximal to the articulable portion. When in the first position, a longitudinal axis passing through the articulable portion may be aligned with a longitudinal axis of the proximal portion of the elongated shaft assembly. Correspondingly, when in the fully articulated configuration, the distal tip of the elongated shaft assembly, and the longitudinal axis of the articulable portion, is oriented at an articulation angle relative to the longitudinal axis of the proximal portion. In the fully articulated configuration a substantial portion of the edges of cutouts along the device shaft are in contact with each other. In one embodiment, the articulation angle of the fully articulated configuration may be between 15 degrees and 90 degrees though it should be understood that the current disclosure is not limited to any particular range of articulation angles. Moreover, in some embodiments, the articulable portion may be movable to one or more additional articulated positions between the non-articulated (i.e., straight) configuration and the fully articulated configuration.

The surgical devices described herein may be made out of any desirable material or combination of materials. In some instances, the surgical devices described herein may be made from materials that are either sterilized and/or are sterilizable using any appropriate method including, but not limited to, heat, radiation, and/or pressure. Moreover, the materials may be capable of either being sterilized before, during, or after assembly and packaging to maintain sterility.

In some embodiments, a surgical instrument may include an elongated shaft assembly including a first articulating shaft and a second articulating shaft coaxially arranged relative to the first articulating shaft. The first and second articulating shafts may include flexible portions that form an articulable portion of the elongated shaft assembly, and the first and second articulating shafts are axially fixed relative to one another at a location distally located relative to the articulable portion. Proximal portions of the first and second articulating shafts may be axially displaceable relative to one another to move the articulable portion of the elongated shaft assembly between the first and second positions. For example, the proximal portions of the first and second articulating shafts may be displaced relative to one another to selectively place the first and second articulating shafts in opposing states of tension and/or compression. As discussed in more detail below, such tensile and/or compressive forces may be transmitted through a suitable structure in the articulable portion to apply and/or release a bending moment in the first and second articulating shafts, thereby moving the articulable portion between the non-articulated and articulated configurations. In some embodiments, the bending moment causes the articulable portion to move from the non-articulated configuration, which may correspond to a relaxed configuration of the elongated shaft assembly, to the articulated configuration. However, it should be understood that the current disclosure is not limited to embodiments in which a bending moment causes movement towards an articulated configuration. For example, in some embodiments, the fully articulated configuration may correspond to a relaxed (i.e., stress-free) state for the elongated shaft assembly, and application of a bending moment (or other suitable stresses) may cause the elongated shaft assembly to move toward the non-articulated (i.e., straight) configuration.

In some embodiments, a surgical instrument may include an articulation control operable by a user to selectively move an articulable portion of an elongated shaft assembly of the device between non-articulated and fully articulated configurations. The articulable portion of an elongated shaft assembly may be formed by one or more flexible portions of the associated shafts that permit articulation. For example, the flexible portions of the shafts may include a plurality of cutouts extending substantially in a transverse direction across a diameter of the shafts and arranged along at least a portion of the length of the various shafts comprising the elongated shaft assembly to provide a desired flexibility. It may be beneficial to provide a desired rigidity of the elongated shaft assembly while still permitting articulation of an articulable portion of the elongated shaft assembly. Accordingly, in some embodiments, the specific dimension and arrangement of the cuts, spines, and/or other suitable features of at least first articulating shaft of the elongated shaft assembly may be selected to provide the desired stiffness. In one embodiment, the spines may have a tapered configuration with distal portions of the spines being narrower than proximal portions thereof. This may provide an increased bending stiffness of the elongated shaft assembly at a proximal end of the spines and increased flexibility of the assembly at the distal end. Such a configuration may permit the distal end of the articulating shaft assembly to have enough flexibility to articulate to a desired articulated position while also becoming progressively stiffer at the proximal end of the articulable portion. Without being bound by theory, such a configuration may help to avoid undesired deflection of an elongated shaft assembly during use, for instance, when a user presses a distal end of the shaft assembly against a surface to deploy a fastener into tissue.

In addition to the above, the inventors have recognized that the number, size, and/or spacing of the cuts in the shafts of an articulable portion of an elongated shaft assembly may influence the resulting stiffness of the elongated shaft assembly in the non-articulated and/or articulated configurations. For example, the inventors have found that articulating shafts having increased numbers of cuts and smaller cut sizes in the articulable portion may provide for enhanced stiffness while still permitting a desired amount of articulation of the articulable portion. Accordingly, in some embodiments the number of cuts, the cut size, and/or cut spacing may be selected to provide a desired stiffness for the elongated shaft assembly. Specific sizings and spacings of the cuts are discussed in more detail below in regards to specific embodiments.

As noted above, an elongated shaft assembly may include first and second articulating shafts that are placed in opposing states of tension and compression when an articulable portion of the elongated shaft assembly is in an articulated configuration. In some embodiments, the articulating shaft that is placed in the compressive state may include a plurality of cuts that are sized and shaped such that opposing sides of each of the cuts come into contact with one another when the articulable portion is fully articulated. For example, the inventors have appreciated that such configurations may impart additional stability and/or rigidity to a distal portion of the elongated shaft assembly when in the articulated configuration.

Turning to the figures, specific non-limiting embodiments are described in further detail. It should be understood that the various systems, components, features, and methods described relative to these embodiments may be used either individually and/or in any desired combination as the disclosure is not limited to only the specific embodiments described herein.

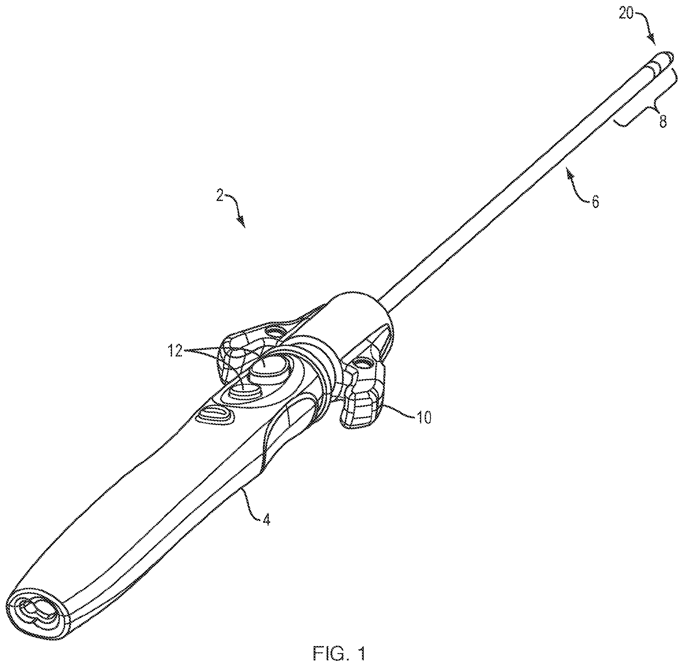

FIG. 1 depicts one embodiment of a surgical instrument 2. The surgical instrument 2 includes a handle 4 and an elongated shaft assembly 6 extending distally from the handle toward a distal end 20. Distal end 20 may provide a variety of functions including but not limited to either a light source, visualization, energy based tissue treatment such as an electrosurgical device, tissue grasping or a fastener may be deployed. The elongated shaft assembly 6 includes an articulable portion 8 that may be moveable between a non-articulated position, and one or more articulated (i.e., curved or bent) positions. A non-articulated position may define a straight configuration of the articulable portion 8. Articulation of the articulable portion 8 may be controlled by an articulation control 10, such as a rotatable and/or axially displaceable knob, handle, lever, or other interface that may be moved or pressed. Articulable portion 8 may angularly offset the distal end 20 from the non-articulated configuration to a plurality of offset angles. The surgical instrument 2 may also include other controls or triggers 12 for grasping tissue, actuating a fastener deployment system or activating an energy based treatment for example.

The articulable portion 8 of the elongated shaft assembly may be moved between at least a first position, such as an unarticulated position, and second position, such as a fully articulated position, using articulation control 10. In this example, control 10 may be rotated around the longitudinal axis of the instrument 2. Depending on the embodiment, the articulable portion 8 may be moved to one or more preselected articulation angles, or the articulable portion 8 may be adjusted to one or more arbitrary (i.e. not preselected) articulation angles.

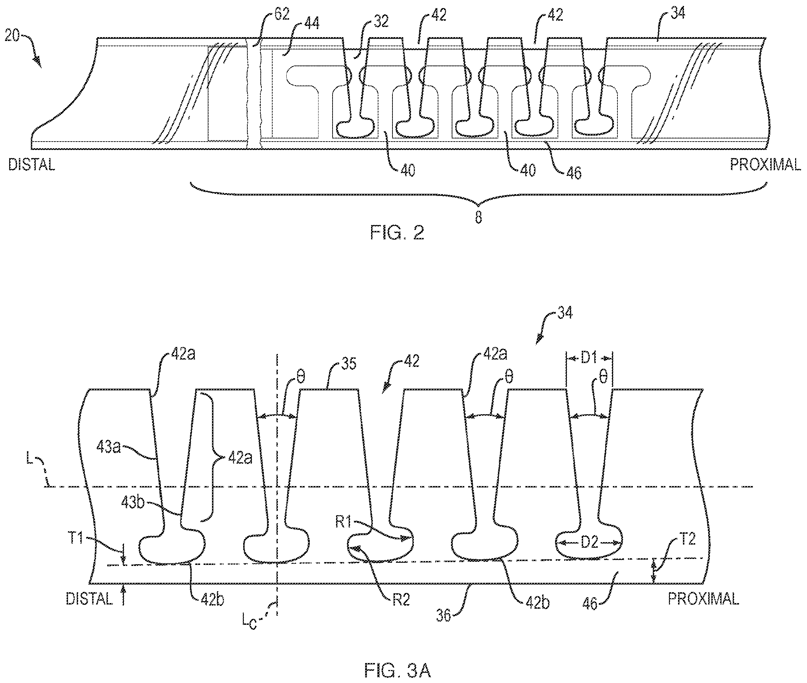

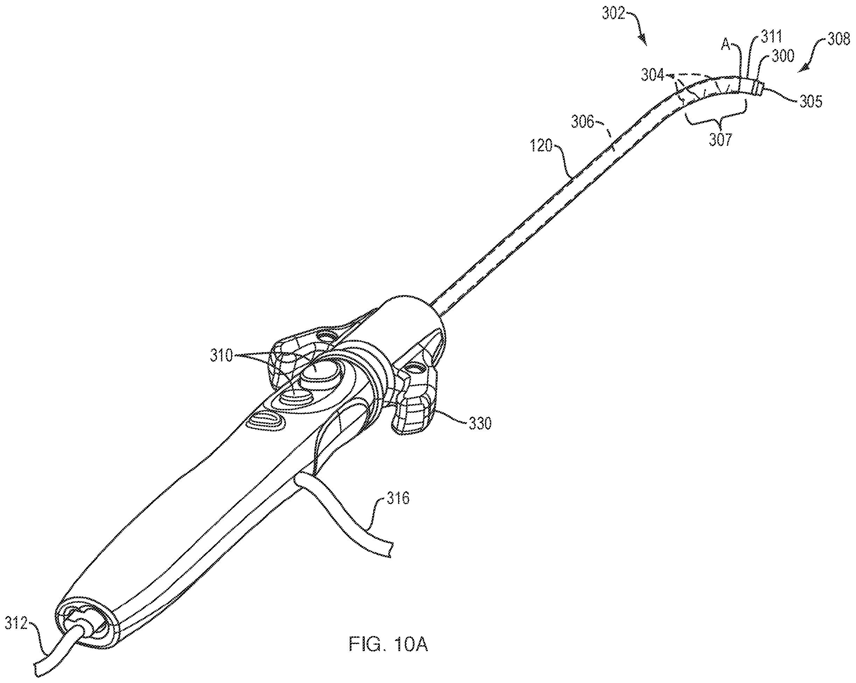

FIG. 2 depicts a side view of the distal end 20 including the articulable portion 8 of the elongated shaft assembly 6 of the surgical instrument 2, which extends distally from the handle 4. The distal end 20 may include other elements, such as sheath, wires, cables or rods external or internal to the shaft assembly, these elements removed from the figure to avoid unduly complicating the figure. Distal end may include electrodes, tubing or grasping jaws that may extend from distal end, also removed from the figured to avoid complicating the figure. For example, outer shaft 34 may define a return electrode and therefore is electrically conductive. An isolated active electrode of an electrosurgical instrument may be coupled to a distal-most end of the outer shaft 34, as will be disclosed in more detail in FIGS 10A-10C.

The elongated shaft assembly may include an inner shaft 32 and an outer shaft 34, the inner shaft 32 extending within the outer shaft 34. As described herein, the shafts (32, 34) are constructed and arranged to move the articulable portion 8 between the non-articulated position and the one or more articulated positions. Shafts (32, 34) of the elongated shaft assembly may be arranged coaxially relative to one another and both shafts may define an elongate lumen along their respective axes, at least along the articulable portion. Although a particular arrangement of shafts is shown in the figures, it should be understood that other arrangements also might be suitable. For example, in an alternative embodiment, the inner shaft 32 may be a flexible pull rod with no lumen or cutouts.

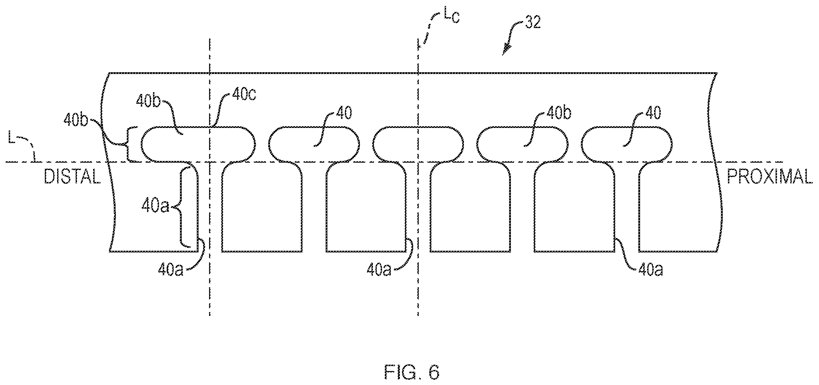

As illustrated, the articulable portion 8 may include a plurality of cutouts (40, 42), which define one or more spines extending along a length of the shafts (32, 34). Seen both in FIG. 2 and FIG. 6, the inner shaft 32 may include a first plurality of cutouts 40 extending in a transverse direction through the shaft 32. The first plurality of cuts 40 are axially spaced from one another along a length of the articulable portion 8 on a first side of the shaft 32. A second opposing side of the shaft 32 may have a continuous, uninterrupted length, defining a spine 44. Similarly, shown in at least FIG. 2 and 3A, the outer shaft 34 includes a plurality of cutouts 42 extending generally orthogonal to the shaft longitudinal axis from a first side of the shaft 34. Cutouts 42 are axially spaced from one another. A second opposing side of the shaft 34 may define a continuous, uninterrupted length, defining a spine 46. The cutouts and spines of each shaft may be arranged on opposing sides of the articulable portion 8 to each other.

The inner and outer shafts 32 and 34 may be attached to one another at an attachment point 62, which is preferably located distally from the articulable portion 8. This may axially fix the inner and outer shafts to one another at the attachment point 62. In the depicted embodiment, the attachment point 62 is located adjacent a distal end of the inner shaft 32, and the outer shaft may extend distally further than the inner shaft 32. The two shafts 32, 34 may be attached in any suitable manner, such as with an adhesive, one or more fasteners, one or more pins, one or more welds, and/or any other appropriate form of connection.

Due to the distal attachment 62 of the two shafts 32, 34, application of axial forces and/or displacements to corresponding proximal portions of the inner and outer shafts 32, 34 may place these shafts in a state of tension and/or compression. For example, a proximally directed force and displacement applied to a proximal portion of the inner articulating shaft 32 may create a tensile stress in the inner shaft 32. Similarly, application of a corresponding distally directed force and displacement to a proximal portion of the outer shaft 34 may create a compressive stress in the outer articulating shaft. These opposing tensile and compressive stresses may be transmitted through the opposing spines 44 and 46 of the respective shafts, which are both offset from a neutral bending axis of the overall elongated shaft assembly. This creates a bending moment in the articulating shafts, which causes the articulating shafts to bend and move the elongated shaft assembly toward an articulated position.

Proximally and distally directed forces and displacements may be applied to the shafts, respectively, via any suitable articulation control system. For example, a surgical instrument may include an articulation control 10 to selectively move an articulable portion 8 of an elongated shaft assembly between the non-articulated and articulated positions. Depending on the particular embodiment, the articulation control may be coupled to articulating shafts (32, 34) of the elongated shaft assembly via any suitable structure to control the articulation. For example, control 10 may be operatively coupled to the inner shaft 32, and actuation of the control 10 may axially retract the inner shaft 32 proximally, while the outer shaft 34 remains fixed and stationary.

Now moving on to FIG. 3A, showing only the outer shaft 34 in a neutral configuration, that may be a straight configuration. Inner shaft 32 is removed to avoid unduly complicating the figure. With reference to articulation, and the resultant bend of the shaft when articulated, outer shaft 34 defines an inner radial side 35 and outer radial side 36 (spine) of the resultant bend. Upon articulation the corresponding edges of each cutouts 42 move towards each other and engage each other and the inner radial side 35 changes from a length of the shaft with cutout openings therealong to a continuous smooth uninterrupted surface (when fully articulated). The opposing outer side 36 of shaft includes a spine 46 and therefore may have no cutouts. While a side view of the shaft 34 is shown throughout these figures, each cutout 42 extends through the tubular shaft such that the cutouts are mirror image relative to a vertical plane along the longitudinal axis L.

Each of the outer shaft cutouts 42 may define a keyhole shape having a longitudinal axis L_{c} transverse the longitudinal axis L of the shaft 34. The keyhole shape includes a tapered or angled opening portion 42a extending from the inner-side 35 and terminating with a rounded or bulbous closed end 42b. Closed end 42b may also be described as a kidney bean shaped end 42b. End 42b is configured to isolate the bending stress to one point within each cut 42. Angled opening portion 42a defines a linear tapered opening, tapering to a smaller opening as the opening portion 42a extends away from inner radial side 35 and towards the rounded end 42b. Keyhole shaped cutout 42 is not symmetrical about the longitudinal axis L_{c}. Keyhole shaped cutout 42 is not mirror image about a vertical plane normal to the instrument longitudinal axis L and extending along longitudinal axis L_{c}. Angled opening 42a is defined by a distal edge 43a and proximal edge 43b that may be linear. Angled portion 42a may taper uniformly and may taper at angle θ that is split evenly either side of axis L_{c}. Angled opening defines a longer distal edge 43a than the proximal edge 43b. The distal edge length of tapered opening 42a may extend further in a direction traverse the longitudinal axis L and thereby remove a larger arc segment of the tubular shaft annular wall. For example, with reference to the view shown in FIG. 3A, for a tubular shaft outer diameter of about 0.2 inches, a distal edge may extend approximately 0.117 inches through this example tubular shaft in a traverse direction to the longitudinal axis of the shaft, while the proximal edge may extend approximately 0.110 inches. The difference between the two edge lengths may depend on the number of cutouts, the bend angle of the fully articulated position, and the angled opening size. The larger the angled opening, the larger the difference in the proximal and distal lengths may be. Each cutout 42 is further asymmetrical in that, the kidney bean shaped end defines a tighter concave curve R2 on the distal side and a larger concave curve R1 on the proximal side. For example, a proximal radius R1 may be approximately 0.0112 inches, while the distal radius R2 may be approximately 0.010 inches. Again, the difference in the two radii (R1, R2) depending on the number of cuts, the bend angle of the fully articulated position, and the angled opening size. The larger the angled opening 42a, the larger the difference in the two radii. The asymmetry in both the edge lengths and radii, is configured to place opposing edges 43 in aligned engagement when in the fully articulated configuration, and thereby improve rigidity of the assembly in the fully articulated configuration, as will be explained in more detail in alter figures.

For the SI units transformation 1 inch is equal to 25.4 mm.

Each cutout 42 defines a maximum opening width D1 along the side 35, defined in the least articulated orientation, which may be a straight configuration. Rounded closed end 42b defines a maximum axial dimension D2. D2 may preferably be larger than D1. However, so as place a plurality of cutouts 42 in close succession, and evenly distribute the articulated curve along the curve length, D2 is preferably less than 50% greater than D1. As shown, D2 is approximately 30% larger than D1. For a shaft that is 0.20 inches in outer diameter, D2 may be approximately 0.052 inches, while D1 may be 0.04 inches for example.

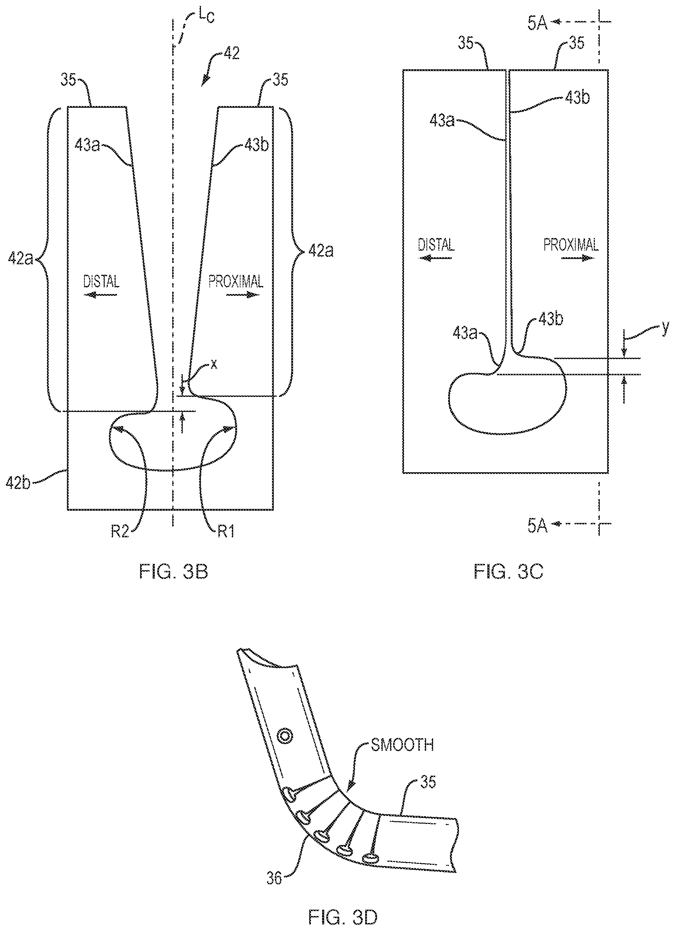

FIG. 3B shows a close up of a profile of a single cutout 42 of the plurality of cutouts 42. The distal and proximal edges (43a, 43b) having different lengths, defining an offset X at a transition to the respective bulbous end 42b. Offset X is defined by the number of cutouts 42 and angled opening dimensions. Offset X may range from about 0.003 inches to 0.015 inches for a 0.15-0.26 inch diameter tube of a surgical device. In addition, as disclosed herein, distal concave surface of rounded end 42b defines a tighter curved side than a corresponding proximal concave surface. Represented in FIG. 3C, once articulated to a fully articulated configuration wherein the two opposing edges 43a and 43b rotate towards each other, this offset X is configured to align edges 43a 43b, so that they abut and align with each other over a longer circumferential or arc length. This forms a continuous smooth inner radial surface 35 when in the fully articulated configuration (seen best in FIG. 3C and 3D). Having this aligned contact along the edges 43 improves rigidity of the assembly in the fully articulated configuration.

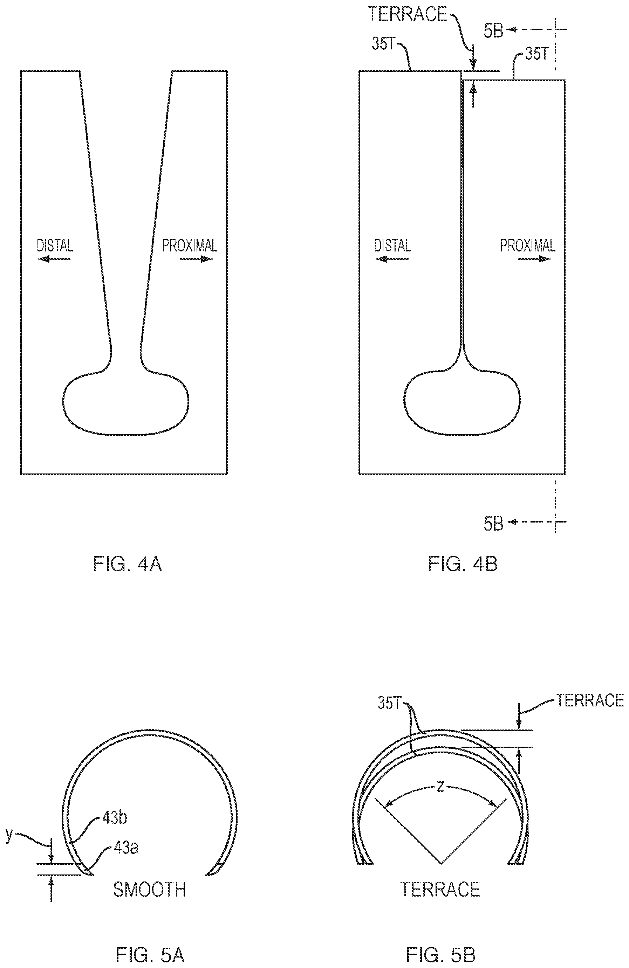

As way of comparison, FIG. 4 represents a resultant shape of the fully articulated configuration should the keyhole profile be symmetrical and absent offset X. In this example, articulation would form an inner surface that is terraced or stepped 35T and would therefore not be a smooth continuous inner curved surface. Eliminating or reducing this terracing 35T increases the torsional stiffness of the device when in the fully articulated configuration. This can be best explained while looking at a comparison of cross sectional illustrations, represented in FIG. 5A and 5B. Shown in FIG. 5A are the two edges 43a, 43b (proximal and distal of an example cutout) of the asymmetrical cutout 42 as disclosed herein (reference FIG. 3C). The bulbous portion 42b is removed to simplify the figure. FIG. 5A illustrates the two edges 43a and 43b aligned with each other, once rotated to a fully articulated position, such that the two edges 43am 43b are directly opposed. FIG. 5B shows two edges of a symmetrical cutout rotated to a fully articulated position with the terrace as illustrated in FIG. 4. While in profile, as shown in FIG. 4 the offset (terrace) may appear similar in length to Y. However, it can be seen when viewed as a cross section in FIG. 5B, that the arc length of contact between the two edges is significantly different between the two figures (FIG 5A and 5B). In FIG, 5A with the two edges 43 of the asymmetrical cutout 42 in a fully articulated configuration, the dimension Y represents a length of the edge 43a not in direct contact with edge 43b. Compare this to the illustration in FIG. 5B representing two circumferential edges of an example cutout of a symmetrical keyhole profile such as that shown in FIG. 4. As shown, the arc length Z represents an arc length of non-aligned edge contact for the symmetrical cutout. Length Z is considerable longer than length Y. The asymmetrical cutout 42 profile is configured to allow for a greater arc length of direct edge 43 contact of each cutout 42. By capitalizing on this edge contact, the articulating shaft can achieve comparable torsional rigidity when fully bent to that of a fixed wand.

Another key characteristic shown in FIG. 3A is the gradual decrease in the width of the shaft's spine from the most proximal cut to the most distal cut. Best seen in FIG. 3A, spine 46 of outer shaft 34 is tapered in thickness T, defined by the overall length of each of the keyhole cutouts 42. Spine 46 grows thicker as the spine extends proximally. T2 is greater than T1. This disperses the stresses more evenly across each of the cuts 42 and allows the shaft 34 to straighten back to its original shape instead of forming a kink along the spine. This is important for the shaft to be able to slide back out of the 5.0 mm cannula after use. The number of cutouts is also refined to disperse stresses but also maintain a tight bend radius that is compatible with human anatomies. A range of between 2-10 cutouts is envisioned by the inventors. A finer more delicate instrument may have more cutouts, each cutout having a smaller angular opening θ, to better distribute the stresses and reduce fatigue along the tube spine.

Furthermore, the angles (θ) of each cutout 42 may be different from each other. In some instances, there may be stress concentrations in the proximal-most and distal-most cutouts. Therefore the angle (θ) is the proximal-most and distal-most cutouts 42 may be smaller than the intermediate cutouts 42. The bend angle in those proximal-most and distal-most cutouts 42 is reduced and the middle cuts are widened to maintain the same overall bend angle of the shaft when fully articulated. Furthermore reducing the angle (θ) of the most distal cutout 42 is also useful because it can be difficult to fully close due to its proximity to the weld point 62. It is preferable that all of the cutouts must completely close when fully articulated to have edge on edge or in most cases metal on metal contact to maximize its torsional rigidity. Therefore, the plurality of cutouts are configured for a specific target bend angle of the fully articulated portion for maximum stiffness and torsion resistance. For example, these geometries were developed for shafts with maximum angles between 30 degrees and 70 degrees. A 30-40 degree shaft may mimic a fixed angle device, as described herein. The 70-degree shaft may be used as a continuously articulating device, meaning that the surgeon may often change the degree of articulation during use.

Returning to FIG. 2 and FIG. 6, the inner shaft 32 is illustrated, with the outer shaft 34 removed from the FIG. 6 for clearer understanding. Inner shaft 32 includes cutouts 40 having an opening portion 40a that may be have two parallel edges defining a uniform width opening along its length up until rounded slots 40b. Rounded slots 40b may also include a straight surface 40c, that may be parallel to the shaft longitudinal axis L-L. These slots 40b create two points of stress concentration in each cut 40 so that the stresses caused by extending the inner shaft are spread out over twice as many locations. The stress dispersion in the inner shaft 32 is important because it undergoes more fatigue by being extended when the outer shaft is bent. Inner shaft cutouts 40 may be symmetrical about the longitudinal axis of each cut L_{c}. A distal most of the inner cuts 40 however may be non-symmetrical about longitudinal axis L_{c}, extending further along the shaft longitudinal axis L distally than proximally.

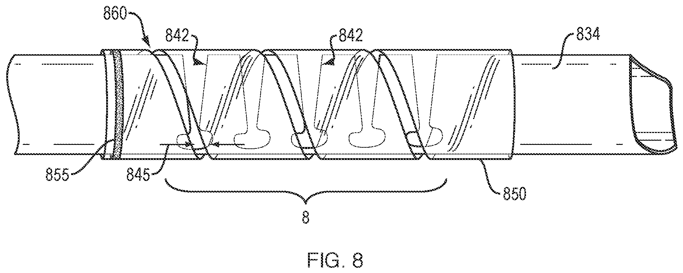

In some embodiments, articulable portion 8 may also include a coiled or slotted liner 850, as shown in at least FIG. 8-10C, sandwiched between the outer sheath 120 and outer shaft 834. Slotted liner 850 may limit migration of an outer sheath 120 of an articulating device between openings in the articulable portion 8, and as such may be applicable to any articulable shaft with cutouts that also requires an outer flexible sheath as part of its construct.

FIG. 8 shows a distal portion of an outer tube 834 that includes cutouts 842. Outer tube 834 may be similar to outer tube 34. Cutouts 842 may be similar to cutouts 42. Sheath 120 is absent in FIG. 8 for clarity of understanding of liner 850. Sheath 120 coaxially covers liner 850 is shown. Liner 850 may extend over and along the outer shaft 834. Liner 850 may extend beyond a proximal most and distal most cutout 842 and may define a flexible sleeve. Liner 850 may be fixedly coupled to tubular member 834 via adhesive for example. However, since the liner 850 is preferably flexible to avoid hindrance of articulation of the shaft 834, small discrete areas of fixation are preferable, rather than over a broad surface of the liner 850 that may unduly add rigidity. For example, the liner 850 may be fixedly coupled in 1-3 spots or along a narrow circumferential line 855 for example around the liner surface. In some embodiments liner 850 may be constrained from slipping along the shaft 834 by assembly of sheath 120 alone. Liner 850 may be fixedly coupled at one end only, to allow the liner 850 to axially move during instrument articulation.

As explained earlier, a sheath 120 over an articulable portion 8 may be required as part of the instrument's function, for example to electrically isolate a shaft and/or limit debris and ingress through the cut outs 842. However, the sheath 120 is preferably flexible to minimally influence articulation and this flexibility may inherently allow migration of the sheath 120 into the cutouts 842. Therefore, the inventors have developed a liner 850 for placing coaxially between the shaft 842 and sheath 120 to limit sheath migration. The liner is a thin sleeve to evade adding bulk or diameter to the device, and has a stiffness configured to resist migration of both itself and the sheath 120 into the cutouts 842. Making the liner stiffer to avoid migration however may hinder articulation as disclosed herein. Therefore, the liner 850 includes discontinuities in the form of slots or cuts through the liner thickness, configured to increase the liner's flexibility during articulation. Slots or cuts may be orientation to maintain a barrier to sheath migration into cutouts 842. Slots or cuts may be orientated at a non-zero angle to both a longitudinal axis and transverse axis of the liner 850. In some embodiments sheath may be a heat shrink, and therefore the process of shrinking sheath may naturally form sheath 120 to the shape and cuts along liner 850.

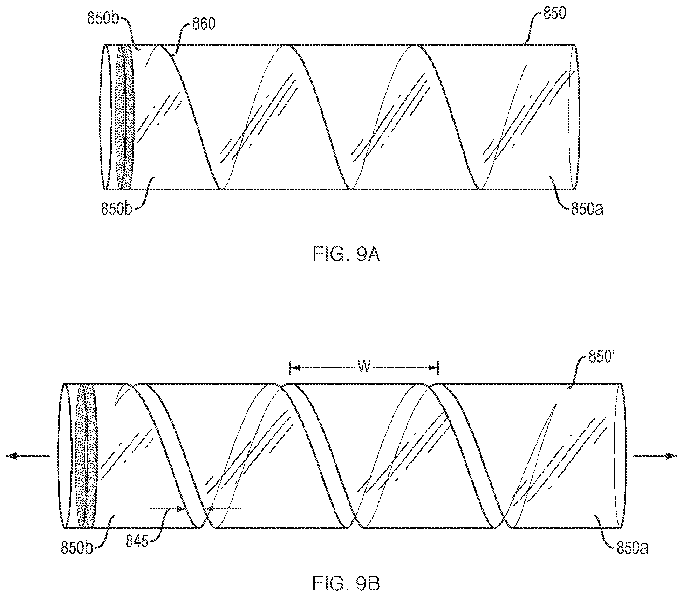

As shown in FIG. 9A and 9B, showing only the liner, the slots or cuts may be a continuous helical cut 860 along a length of the liner 850 to increase flexibility. The sheath 120 preferably does not include these discontinuities as this would alter the reason for the sheath 120. For example, a sheath 120 with cuts is no longer an effective electrical isolator or fluid barrier. Liner 850 may therefore define a section of thin flexible tubing that may be nylon and may include a helical or spiral cut 860. The liner 850 preferably covers the articulable portion 8, including a proximal most and distal most cutout 842. The helical cut 860 may extend along a medial portion of the liner 850, leaving both ends (850a, 850b) of the liner free of cuts and thereby tubular ends free of discontinuities. These cut-free ends (850a, 850b) may be about 0.1-0.3 inches in axial length. Liner 850 may cover and be coaxial with the articulable portion 8 only and be entirely spaced away from handle of instrument. Liner 850 does not provide force transfer means and thereby may not directly control articulation of the device. Liner 850 is configured to act as a partial barrier and at least partially block entrance of sheath 120 into articulable portion 8. The liner 850 may be placed over the plurality of axially spaced cutouts 842 of the articulable portion 8 and in the straight (non-articulated) configuration the liner 850 may be axially spread (as shown by arrows in FIG. 9B) to create axial gaps 845 along the liner 850 in an axially stretched form 850'. Both ends 850a and 850b may then be fixed to shaft 834 in this stretched form 850'. The inventors have found that forming these gaps 845 increases flexibility of the liner 850 during articulation. In alternative embodiments, gaps 845 may be formed by removing material. In other words, by forming two parallel cuts in a spiral and thereby removing material equal to the width of the gap 845. The width and spacing of the spiral cut(s) 860 may be configured to both reduce exposure of the insulation sheath 120 to the radial cutouts 842 while also minimally altering articulation of the device. More specifically spiral cut and gaps 845 are configured to add flexibility to the liner 850 and thereby reduce any resistance this liner may add to shaft articulation. In further alternative embodiments, the spiral cut may not be spread to form gaps 845 while in a straight non-articulated position. This may depend on the flexibility of the liner 850 material, and angular offset value of articulation. For example, the lesser the angular offset a device is configured to articulate, the less flexible the liner 850 may need to be and the axial gaps may not be necessary. Therefore, gap 845 may be negligible until the shaft is articulated, whereupon the gaps 845 may expand or contract accordingly. Larger axial gaps 845 along the spiral cut may reduce any resistance to shaft articulation, but may also increase exposure of the radial cutouts 842 to sheath 120 and risk interference between sheath 120 and cutouts 842.

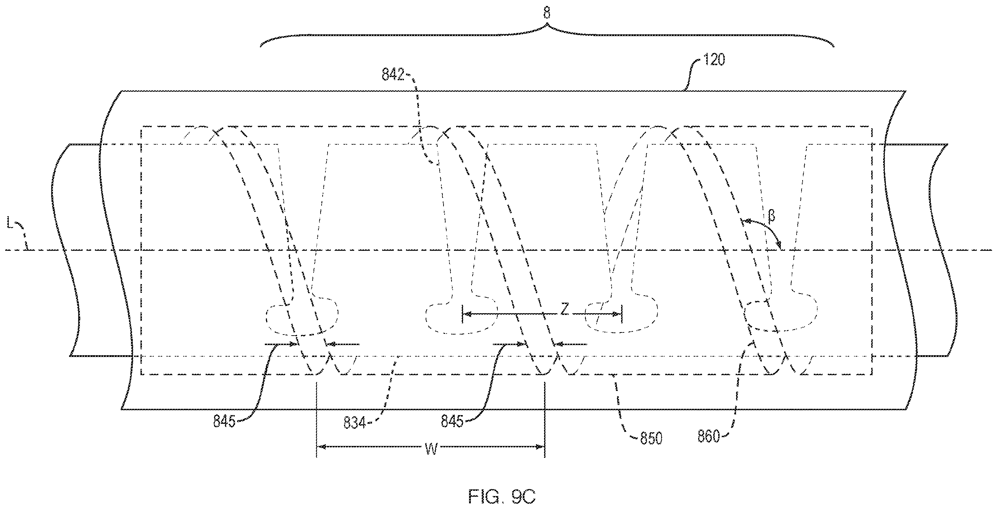

While the inventors envision a range of spiral cut angles, widths and spacings, in order to reduce exposure to the cutouts 842, spiral cut 860 may preferably be defined by the axial distance along the liner 850 each full revolution the spiral cut 860 makes, which is indicated as distance W in FIG. 9B and FIG. 9C. More specifically the spiral cut 860 may be preferably defined in that substantially all of each revolution of the spiral cut 860 about the length of the liner 850 occurs within a respective distance along the length of the articulable portion 8 of at least an axial distance between two successive cutouts 842. More preferably, the spiral cut may be defined in that substantially all of each revolution of the spiral about the length of the liner 850 occurs within a respective distance along the length of the articulable portion 8 of at least an axial distance (Z) between two successive cuts 842. The distance W is preferably greater than the distance Z.

FIG. 9C schematically represents an example embodiment of the articulating shaft 834 with liner 850 and sheath 120, with the shaft 834 in a straight configuration. Tubular shaft 834 includes a plurality of example axially spaced cutouts 842 at least partially covered by liner 850. Liner 850 may be a thin flexible sleeve or tube with angled or spiral cuts 860 extending along a length of the liner 850. Angled cuts, or spiral cut 860 may extend along an axial length approximately equivalent to an axial length of articulating portion 8. Spiral cut 860 may be axially spread to form small gaps 845 to increase flexibility of liner 850. Spiral cut 852 may define an axial length of each spiral "W". Spiral cut 852 may be formed at angle β relative to the longitudinal axis L, angle β ranging from 90-180 degrees, and may more preferably range between 100-170 degrees. In this example cross section, the spiral cut 860 may be defined in that substantially all of each revolution of the spiral about the length of the liner 850 occurs within a respective distance along the length of the tubular shaft 834 greater than an axial distance between a first radial cutout to a directly adjacent radial cutout, indicated as Z on FIG. 9C.

Advantageously this liner 250 may reduce migration of a sheath in an electrosurgical instrument wherein the electrical energy is insulated by a sheath along portions of the instrument. Instrument may be monopolar or bipolar and may be used to treat tissue using energy such as but not limited to cutting, coagulating, ablating and heating. An example electrosurgical instrument may include a Coblation wand, such as a wand described in US Patent 10,420,601.

FIG. 10A shows an example wand and more specifically an elevation view of an example electrosurgical wand 302 in accordance with example systems. In particular, wand 302 comprises elongate shaft 306 that may be similar to shaft 34 or shaft 834, disclosed in earlier figures. Shaft 306 may define a tubular member for receiving electrical wires therealong, and fluid aspiration or fluid delivery tubing therethough and may include a series of radial cutouts 304 defining an articulable portion 307 of the shaft 306. Wand 302 may also include a handle 310 coupled to the proximal end of the elongate shaft 306, the handle 310 including actuation controls 330 to articulate the articulable portion 307. Also visible in FIG. 10A is a flexible tubular member 316 extending from the handle 310 and the multi-conductor cable 312. The wand 302 may comprise an active electrode 305 disposed on the distal end 308 of the elongate shaft 306. Active electrode 305 may be coupled to an active or passive control network within a controller (not shown) by means of one or more insulated electrical connectors (not shown) in the multi-conductor cable 112. Active electrode 305 is isolated from the shaft 306 by an electrode support member 300 coupled to the distal end of elongate shaft 306. The active electrode 305 may be electrically isolated from a common or return electrode, which is disposed on the shaft proximally of the active electrode 302, in some example systems within 1 millimeter (mm) to 25 mm of the distal tip. The return electrode 311 may define a distal end of shaft tubular member 306 and may have a perimeter at least partially defined by a coaxial sheath 120 that terminates at "A" and thereby defines an exposed electrically conductive portion of tubular member sized and configured to operate as a return electrode 311. The support member 300 is positioned distal to the return electrode 311 and may be composed of an electrically insulating material such as epoxy, plastic, ceramic, silicone, glass or the like. Support member 300 extends from the elongate shaft 306 (usually about 1 to 20 mm) and provides support for active electrode 305. Of note for clarity of discussion FIG. 10A does not show liner 850, as it is covered by sheath 120 in this figure. In addition, for clarity of discussion, FIGS. 10A-10C do not show a mechanism of articulating the articulating portion 307 to not unduly complicate the figure. As an example mechanism (not shown) an inner shaft or pull rod may extend along shaft 306 and may be coaxially arranged with tubular member and axially fixed at an attachment point located distally from the articulable portion 307. Proximal portions of the inner shaft may be displaceable along the longitudinal axis of shaft 306 to articulate the articulable portion 307 from the non-articulated configuration (or less articulated configuration) to the articulated configuration. Inner shaft may be an inner tubular member, and may also include a plurality of axially spaced radial cutouts to facilitate articulation, along the articulating portion 307 that may be on a diametrically opposing side relative to radial cutouts 304, similar to the embodiments disclosed herein.

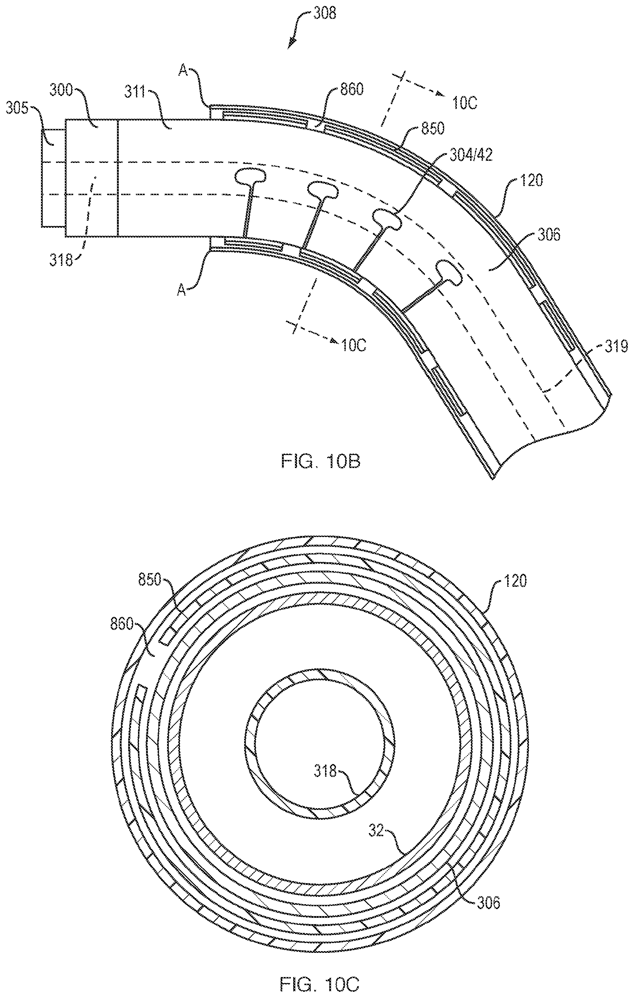

FIG. 10B shows a distal end 308 of the wand 302 in accordance with example embodiments, with the sheath 120 and liner 850 partially removed. In particular, wand 302 may comprise a fluid conduit, such as a suction lumen 318 extending along the elongate shaft 306, through the spacer 300 and electrode 305. In some embodiments, the inside diameter of the tubular member 306 may define the suction lumen 318. As shown a separate tubing 319 within the elongate shaft 306 may define a portion of the suction lumen 318. The suction lumen 318 may provide a conduit for aspirating excess fluids, bubbles, tissue fragments, and/or products of ablation from the target site proximate to the active electrode 302. In the embodiments where the shaft 306 provides a portion of suction lumen conduit, sheath 120 may prevent inadvertent aspiration through cutouts 304, 842 or 42. Suction lumen 318 extends into the handle 310 and fluidly couples to the flexible tubular member 316 that maybe coupled to a peristaltic pump (not shown).

FIG. 10C shows a perspective overview of the distal end 308 of wand 302, in accordance with example systems. In particular, active electrode 305 may be an active screen electrode. Screen electrode 305 may comprise a conductive material, such as tungsten, titanium, molybdenum, platinum, or the like. Screen electrode 305 may have a diameter in the range of about 0.5 to 8 mm, in some cases about 1 to 4 mm, and a thickness of about 0.05 to about 2.5 mm, in some cases about 0.1 to 1 mm. Screen electrode 305 may comprise at least one aperture 312 configured to rest over the distal opening 388 of suction lumen 318. Aperture(s) 312 are designed to enable the passage of aspirated excess fluids, bubbles, and gases from the ablation site and are large enough to enable ablated tissue fragments to pass through into suction lumen 318.

FIG. 10C represents a cross section, with referenced in FIG. 10C. A plurality of tubular elements or shafts may be coaxially disposed along the articulable portion 307. As shown, the outermost sheath 120 may define the external surface of device 302 and may be configured to electrically isolate portions of the device. Sheath 120 may be coaxial with and wrap around a liner 850 and may be heat shrunk around the liner 850 to partially mold to the liner form. Liner 850 may include cuts 860, configured to inhibit migration of the sheath 120 into shaft cutouts as explained previously. The liner is coaxially sandwiched between the sheath 120 and an articulating shaft 306. Liner 850 may be fixedly coupled to shaft 306 at discrete locations with adhesives, for example. Shaft 306 may include articulating cutouts 42/304 (shown in other figures). Inner lumen of shaft 306 may be coupled to an inner rod or tube such as tube 32 disclosed herein, and displacement of the inner rod or tube 32 may actuate the articulable portion 307. Inner tube or rod 32 may also include cutouts (not shown in this figure). Electrical cables (not shown) may extend along shaft 306 to provide energy to electrodes. Flexible tubing 319 may extend along the shaft for fluid to flow therethrough. In some embodiments, the inner lumen of the inner tube 32 or the inner lumen of the shaft 306 may form a portion of a boundary of a fluid flow conduit.

The invention is defined by the appended claims.

## Claims

1. A surgical instrument (2) comprising:
a handle (4); and
an elongate shaft assembly (6) extending distally from the handle (4) and defining a longitudinal axis, the elongated shaft assembly (6) configured to articulate between a fully flexed configuration and a lesser flexed configuration, the elongated shaft assembly (6) comprising:
a tubular member (110) including a flexible portion (8) with a first preferential bending direction;
an elongate member (306) coaxially disposed within the tubular member (110) and directly coupled to the tubular member (110) at a location distal the flexible portion (8);
wherein the flexible portion (8) is defined by a plurality of cutouts (40, 42), each cutout (40, 42) defining a cutout longitudinal axis, and wherein each cutout (40, 42) includes a tapered portion (42a) defined by a proximal linear edge (43b) and a distal linear edge (43a); and
wherein at least one of the plurality of cutouts (40, 42) define an asymmetrical shape about the cutout longitudinal axis, the asymmetric shape configured to increase a circumferential length of contact between a corresponding proximal (43b) and distal (43a) linear edge when the flexible portion is in the fully flexed configuration; and
wherein each of the cutouts (40, 42) have a keyhole shape with a bulbous closed end (42b), the bulbous closed end (42b) asymmetrical about the cutout longitudinal axis.

2. The surgical instrument of claim 1 wherein the bulbous closed ends (42b) are defining a proximal concave surface (R1) and a distal concave surface (R2), continuous with each other and wherein the distal concave surface (R2) defines a tighter radius of curvature than the proximal concave surface (R1).

3. The surgical instrument of claim 2 wherein the bulbous closed end (42b) defines a maximum axial length that is less than 50% larger than a maximum axial length of tapered portion (42a) when the instrument is in a lesser flexed configuration.

4. The surgical instrument of claim 1 wherein in the fully flexed configuration a corresponding proximal edge (43b) and distal edge (43a) of each of the plurality of cutouts (40, 42) align with each other to form a continuous inner curved shaft surface (35), free of terracing.

5. The surgical instrument of claim 1 wherein the cutout asymmetrical shape (42) is configured to rotate the distal edge (43a) towards the proximal edge (43b) so that they align once engaged.

6. The surgical instrument of claim 2 wherein the plurality of cutouts (40, 42) each define an angular opening when the shaft is in a less articulated configuration, and wherein a distal-most cutout (40, 42) of the plurality of cutouts has a smaller angular opening than all of the remaining plurality of radial cutouts (40, 42).

7. The surgical instrument of claim 1 wherein the bulbous closed end (42b) defines a concave proximal surface (R1) and a concave distal surface (R2) facing the concave proximal surface (R1) and different to the concave proximal surface (R2).

8. The surgical instrument of claim 7 wherein the concave proximal surface (R1) defines a larger radius of curvature than the concave distal surface (R2).

9. The surgical instrument of claim 7 wherein the asymmetrical shape (42) is configured form a continuous uninterrupted curve along the articulable portion (35, 36) when the distal end (20) is flexed at the maximum offset angle.

10. The surgical instrument of claim 7 wherein the at least one cutout (40, 42) comprises at least two cutouts (40, 42), each of the at least two cutouts (40, 42) defining an angular opening and wherein when the shaft (6) is in a less flexed configuration, the angular opening of the distal-most cutout (40, 42) of the at least two cutouts (40, 42) has a smaller angular opening than all remaining cutouts (40, 42).

## Patentansprüche

1. Chirurgisches Instrument (2), umfassend:
einen Griff (4); und
eine längliche Schaftanordnung (6), die sich distal von dem Griff (4) erstreckt und eine Längsachse definiert, wobei die längliche Schaftanordnung (6) dazu konfiguriert ist, sich gelenkig zwischen einer vollständig gebogenen Konfiguration und einer weniger gebogenen Konfiguration zu bewegen, wobei die längliche Schaftanordnung (6) umfasst:
ein röhrenförmiges Element (110), das einen flexiblen Abschnitt (8) mit einer ersten bevorzugten Biegerichtung einschließt;
ein längliches Element (306), das koaxial innerhalb des röhrenförmigen Elements (110) angeordnet ist und an einer Stelle distal von dem flexiblen Abschnitt (8) direkt an das röhrenförmige Element (110) gekoppelt ist;
wobei der flexible Abschnitt (8) durch eine Vielzahl von Ausschnitten (40, 42) definiert ist, wobei jeder Ausschnitt (40, 42) eine Ausschnittslängsachse definiert, und wobei jeder Ausschnitt (40, 42) einen verjüngten Abschnitt (42a) einschließt, der durch eine proximale lineare Kante (43b) und eine distale lineare Kante (43a) definiert ist; und
wobei mindestens einer der Vielzahl von Ausschnitten (40, 42) eine asymmetrische Form um die Ausschnittslängsachse definiert, wobei die asymmetrische Form dazu konfiguriert ist, eine Umfangslänge eines Kontakts zwischen einer entsprechenden proximalen (43b) und distalen (43a) linearen Kante zu verlängern, wenn der flexible Abschnitt in der vollständig gebogenen Konfiguration ist; und
wobei jeder der Ausschnitte (40, 42) eine Schlüssellochform mit einem bauchigen geschlossenen Ende (42b) aufweist, wobei das bauchige geschlossene Ende (42b) um die Ausschnittslängsachse asymmetrisch ist.

2. Chirurgisches Instrument nach Anspruch 1, wobei die bauchigen geschlossenen Enden (42b) eine proximale konkave Fläche (R1) und eine distale konkave Fläche (R2) definieren, die miteinander durchgehend sind, und wobei die distale konkave Fläche (R2) einen engeren Krümmungsradius als die proximale konkave Fläche (R1) definiert.

3. Chirurgisches Instrument nach Anspruch 2, wobei das bauchige geschlossene Ende (42b) eine maximale axiale Länge definiert, die um weniger als 50 % größer als eine maximale axiale Länge eines verjüngten Abschnitts (42a) ist, wenn das Instrument in einer weniger gebogenen Konfiguration ist.

4. Chirurgisches Instrument nach Anspruch 1, wobei eine entsprechende proximale Kante (43b) und eine distale Kante (43a) jedes der Vielzahl von Ausschnitten (40, 42) in der vollständig gebogenen Konfiguration aufeinander ausgerichtet werden, um eine durchgehende innere gewölbte Schaftfläche (35) zu bilden, die frei von Terrassenbildung ist.

5. Chirurgisches Instrument nach Anspruch 1, wobei die asymmetrische Ausschnittsform (42) dazu konfiguriert ist, die distale Kante (43a) zu der proximalen Kante (43b) hin zu drehen, so dass sie aufeinander ausgerichtet sind, sobald sie im Eingriff stehen.

6. Chirurgisches Instrument nach Anspruch 2, wobei die Vielzahl von Ausschnitten (40, 42) jeweils eine Winkelöffnung definiert, wenn der Schaft in einer weniger gelenkig bewegten Konfiguration ist, und wobei ein am meisten distaler Ausschnitt (40, 42) der Vielzahl von Ausschnitten eine kleinere Winkelöffnung als alle der restlichen Vielzahl von radialen Ausschnitten (40, 42) aufweist.

7. Chirurgisches Instrument nach Anspruch 1, wobei das bauchige geschlossene Ende (42b) eine konkave proximale Fläche (R1) und eine konkave distale Fläche (R2), die der konkaven proximalen Fläche (R1) zugewandt ist und sich von der konkaven proximalen Fläche (R2) unterscheidet, definiert.

8. Chirurgisches Instrument nach Anspruch 7, wobei die konkave proximale Fläche (R1) einen größeren Krümmungsradius als die konkave distale Fläche (R2) definiert.

9. Chirurgisches Instrument nach Anspruch 7, wobei die asymmetrische Form (42) dazu konfiguriert ist, eine durchgehende ununterbrochene Kurve entlang dem gelenkig bewegbaren Abschnitt (35, 36) zu bilden, wenn das distale Ende (20) mit dem maximalen Versatzwinkel gebogen ist.

10. Chirurgisches Instrument nach Anspruch 7, wobei der mindestens eine Ausschnitt (40, 42) mindestens zwei Ausschnitte (40, 42) umfasst, wobei jeder der mindestens zwei Ausschnitte (40, 42) eine Winkelöffnung definiert und wobei, wenn der Schaft (6) in einer weniger gebogenen Konfiguration ist, die Winkelöffnung des am meisten distalen Ausschnitts (40, 42) der mindestens zwei Ausschnitte (40, 42) eine kleinere Winkelöffnung als alle restlichen Ausschnitte (40, 42) aufweist.

## Revendications

1. Instrument chirurgical (2) comprenant :
une poignée (4) ; et
un ensemble d'arbre allongé (6) s'étendant de manière distale à partir de la poignée (4) et définissant un axe longitudinal, l'ensemble d'arbre allongé (6) configuré pour s'articuler entre une configuration entièrement fléchie et une configuration moins fléchie, l'ensemble d'arbre allongé (6) comprenant :
un élément tubulaire (110) incluant une partie flexible (8) avec une première direction de courbure préférentielle ;
un élément allongé (306) disposé de manière coaxiale au sein de l'élément tubulaire (110) et directement couplé à l'élément tubulaire (110) au niveau d'un emplacement distal par rapport à la partie flexible (8) ;
dans lequel la partie flexible (8) est définie par une pluralité de découpes (40, 42), chaque découpe (40, 42) définissant un axe longitudinal de découpe, et dans lequel chaque découpe (40, 42) inclut une partie effilée (42a) définie par un bord linéaire proximal (43b) et un bord linéaire distal (43a) ; et
dans lequel au moins l'une de la pluralité de découpes (40, 42) définit une forme asymétrique par rapport à l'axe longitudinal de découpe, la forme asymétrique configurée pour augmenter une longueur circonférentielle de contact entre un bord linéaire proximal (43b) et un bord linéaire distal (43a) correspondants lorsque la partie flexible est dans la configuration entièrement fléchie ; et
dans lequel chacune des découpes (40, 42) a une forme de trou de serrure avec une extrémité fermée bulbeuse (42b), l'extrémité fermée bulbeuse (42b) asymétrique par rapport à l'axe longitudinal de découpe.

2. Instrument chirurgical selon la revendication 1 dans lequel les extrémités fermées bulbeuses (42b) définissent une surface concave proximale (R1) et une surface concave distale (R2), continues l'une avec l'autre, et dans lequel la surface concave distale (R2) définit un rayon de courbure plus serré que la surface concave proximale (R1).

3. Instrument chirurgical selon la revendication 2 dans lequel l'extrémité fermée bulbeuse (42b) définit une longueur axiale maximale qui est moins de 50 % plus grande qu'une longueur axiale maximale de la partie effilée (42a) lorsque l'instrument est dans une configuration moins fléchie.

4. Instrument chirurgical selon la revendication 1 dans lequel dans la configuration entièrement fléchie un bord proximal (43b) et un bord distal (43a) correspondants de chacune de la pluralité de découpes (40, 42) s'alignent l'un avec l'autre pour former une surface d'arbre incurvée interne continue (35), exempte de terrasses.

5. Instrument chirurgical selon la revendication 1 dans lequel la forme asymétrique de découpe (42) est configurée pour amener le bord distal (43a) à tourner vers le bord proximal (43b) de manière à ce qu'ils s'alignent une fois entrés en prise.

6. Instrument chirurgical selon la revendication 2 dans lequel la pluralité de découpes (40, 42) définissent chacune une ouverture angulaire lorsque l'arbre est dans une configuration moins articulée, et dans lequel une découpe la plus distale (40, 42) de la pluralité de découpes a une ouverture angulaire plus petite que toutes les découpes restantes de la pluralité de découpes radiales (40, 42).

7. Instrument chirurgical selon la revendication 1 dans lequel l'extrémité fermée bulbeuse (42b) définit une surface proximale concave (R1) et une surface distale concave (R2) faisant face à la surface proximale concave (R1) et différente de la surface proximale concave (R2).

8. Instrument chirurgical selon la revendication 7 dans lequel la surface proximale concave (R1) définit un rayon de courbure plus grand que la surface distale concave (R2).

9. Instrument chirurgical selon la revendication 7 dans lequel la forme asymétrique (42) est configurée pour former une courbe ininterrompue continue le long de la partie articulable (35, 36) lorsque l'extrémité distale (20) est fléchie à l'angle de décalage maximal.

10. Instrument chirurgical selon la revendication 7 dans lequel l'au moins une découpe (40, 42) comprend au moins deux découpes (40, 42), chacune des au moins deux découpes (40, 42) définissant une ouverture angulaire et dans lequel lorsque l'arbre (6) est dans une configuration moins fléchie, l'ouverture angulaire de la découpe la plus distale (40, 42) des au moins deux découpes (40, 42) a une ouverture angulaire plus petite que toutes les découpes restantes (40, 42).
